# EUROPEAN PATENT SPECIFICATION

(11) **EP 1 797 828 B1**
(45) Date of publication and mention of the grant of the patent: **24.06.2009**
(21) Application number: 06445074.5
(22) Date of filing: 11.12.2006
(51) Int. Cl.: A61B 17/04, A61B 17/86

(54) **Threaded suture anchor with starting pitch**
Gewindenahtanker mit Anfangssteigung
Ancrage de suture fileté avec pas initial

(30) Priority: 15.12.2005 US 750362 P
(43) Date of publication of application: 20.06.2007
(73) Proprietor: Arthrex, Inc., Naples, Florida 34108-1945 (US)
(72) Inventor: Dreyfuss, Peter J., Naples, Florida 34112 (US)
(74) Representative: Janson, Ronny

(56) References cited:
- WO-A-01/91659
- US-A1- 6 159 235
- US-A1- 2003 069 604

## Description

### BACKGROUND OF THE INVENTION

### 1. Field of the Invention:

The present invention relates to an implant for anchoring surgical suture to bone. More specifically, the present invention relates to a suture anchor having a thread with a starting pitch geometry configured to permit easier insertion into bone.

### 2. Description of the Related Art:

When soft tissue tears away from bone, reattachment becomes necessary. Various devices, including sutures alone, screws, staples, wedges, and plugs have been used to secure soft tissue to bone. Recently, various types of threaded suture anchors have been developed for this purpose.

Suture anchors have been developed and designed to be inserted into a pre-drilled hole. Other suture anchors are self-tapping. Self-tapping screws are shown, for example, in U.S. Pat. No. 4,632,100, which discloses a cylindrical suture anchor. The suture anchor of U.S. Pat. No. 4,632,100 includes a drill bit at a leading end threads spaced from the drill bit for securing the anchor into the hole created by the drill bit. U.S. Pat. No. 5,370,662 discloses a self-tapping suture anchor having a flight of threads around a solid body. U.S. Pat. No. 5,156,616 discloses a similar suture anchor having an axial opening for holding a knotted piece of suture.

US Pat. Appl. No. 2003/0069604 discloses a suture anchor with a continuous thread around a tapering central core, the anchor having an eye for receiving suture.

All of the above-noted suture anchors rely on a flight of threads disposed on the outer surface of a shank to secure the anchor to the bone. They all provide a relatively easy method of suture fixation in hard, cortical bone.

The above-described prior art suture anchors are difficult to secure to the bone, at least initially. For example, following an initial hammering of the suture anchor into the bone, excessive effort may be required to insert the suture anchor along the thread. Use of excessive force may increase the risk of injury to a patient from insertion of the suture anchor especially in softer bone sites, such as cancellous bone, or in bone tissue that has become compromised, such as in osteoporotic bone sites. The structure of cancellous bone is lattice-like, or spongy. Osteoporotic bone is the result of a condition that reduces the quantity of bone or atrophies skeletal tissue, causing a porous condition of the bones. These bone types may present a limited range of specific fixation points that are available to the surgeon.

Accordingly, a need exists for a suture anchor that can be started and secured easily and effectively, especially in softer types of bone.

### BRIEF SUMMARY OF THE INVENTION

The suture anchor of the present invention overcomes disadvantages of the prior art, such as those noted above, by providing a suture anchor according to claim 1 having a thread spiralling helically around a central body and having a configuration that facilitates insertion of the suture anchor into the bone by providing a gradual change from a starting pitch (i.e, a thread disposed along the longitudinal axis of the suture anchor at the distal end of the anchor) to a helical or spiral pitch around the central body. By providing the starting pitch at the distal end of the suture anchor, the suture anchor can be inserted more readily into the bone without the need for additional or excessive force.

The present invention also provides a suture anchor and driver assembly for driving the anchor into bone. Advantageously, the suture anchor of the present invention is provided with threads formed in the shape of a corkscrew, to provide an increased percentage of thread surface area for each turn of the screw, as compared with known suture anchors, thus providing increased pull-out strength, and a decreased tendency for back-out. The driver for driving the corkscrew anchor may be provided with a cleat on the side of the handle. Suture threaded through the cannulated driver can be wrapped around the cleat and fixed for shipping in a slot in the cleat. The suture anchor and driver assembly can be shipped (pre-loaded with suture) as a sterile, surgery-ready unit.

Other features and advantages of the present invention will become apparent from the following description of the invention, which refers to the accompanying drawings.

### BRIEF DESCRIPTION OF THE DRAWINGS

FIG. 1 is a side view of a suture anchor according to the present invention.

FIG. 2 is a proximal end view of the suture anchor of FIG. 1.

FIG. 3 is a distal end view of the suture anchor of FIG. 1.

FIG. 4 is a side view of a suture anchor assembly including a suture anchor, threaded with suture, and a driver according to the present invention.

### DETAILED DESCRIPTION OF THE INVENTION

In the following detailed description, reference is made to various specific embodiments in which the invention may be practiced. These embodiments are described with sufficient detail to enable those skilled in the art to practice the invention, and it is to be understood that other embodiments may be employed.

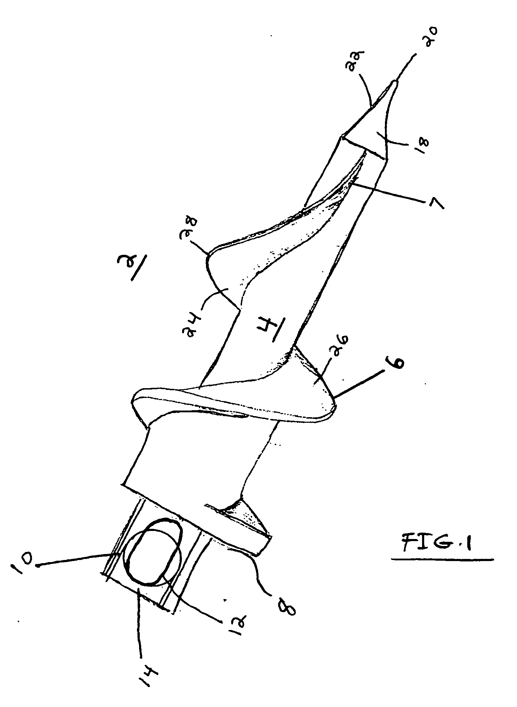

Referring now to the drawings, where like elements are designated by like reference numerals, FIGS. 1-4 illustrate a suture anchor of the present invention. As explained in detail below, the suture anchor of the present invention has a thread spiralling helically around a central body and includes a configuration that facilitates insertion of the suture anchor into the bone by providing a gradual change from a starting pitch (i.e, a thread disposed along the longitudinal axis of the suture anchor at the distal end of the anchor) to a helical or spiral pitch around the central body. By providing the starting pitch at the distal end of the suture anchor, the suture anchor can be inserted more readily into the bone without the need for additional or excessive force.

Advantageously, the threads of the suture anchor of the present invention are formed in the shape of a corkscrew, to provide an increased percentage of thread surface area for each turn of the screw, as compared with known suture anchors, thus providing increased pull-out strength, and a decreased tendency for back-out. The increase in the surface area of the thread is achieved in part by increasing the ratio of the outer diameter of the threads to the inner diameter of the threads. Preferably, the ratio is between 2.25 and 2.75. Most preferably, the ratio of the outer diameter to the inner diameter is 2.5.

Preferably, the suture anchor has a higher thread pitch than prior art screws, thus increasing the area of thread for each turn of the screw, which also leads to greater pull-out strength. Significantly, due to the increased pitch, fewer turns of the corkscrew screw thread are required to advance the suture anchor into position. Accordingly, the suture anchor is easy to install, and displaces less tissue material upon insertion than known suture anchors.

The present invention also provides a suture anchor and driver assembly for driving the corkscrew suture anchor into bone. The driver is formed of a cannulated tube secured to a cannulated handle. A socket (preferably hexagonal) formed on the distal end of the tube holds the suture anchor for rotation and installation into the bone. The outer diameter of the tube is about equal to or less than the outer diameter of the proximal end of the central body of the suture anchor.

The driver is optionally provided with a cleat on the side of the handle so that suture threaded through the cannulated driver can be wrapped around the cleat and fixed for shipping in a slot in the cleat using adhesive foam. One or more sutures, threaded through the suture anchor eye and up through the cannulated driver, can be pulled and secured around the cleat, the suture being pinched under tension. Advantageously, the tensioned suture helps to hold the suture anchor in place at the distal tip of the driver. The suture anchor and driver assembly can be shipped, pre-loaded with suture, as a sterile, surgery-ready unit.

Referring now to the drawings, where like elements are designated by like reference numerals, FIGS. 1-3 illustrate suture anchor 2 of the present invention. As shown in the drawings, the suture anchor 2 includes a body 4 provided in the shape of a tapered cylinder. A continuous thread 6 wraps around body 4 in a clockwise direction in the shape of a corkscrew, as shown. Advantageously, the thread 6 includes a configuration that facilitates insertion of the suture anchor into the bone by providing a gradual change from a starting pitch 7 (i.e, a thread disposed along the longitudinal axis of the suture anchor at the distal end of the anchor) to a helical or spiral pitch around the central body. By providing the starting pitch 7 at the distal end of the suture anchor 2, the suture anchor 2 can be inserted more readily into the bone without the need for additional or excessive force.

The anchor body 4 is preferably formed of a biocompatible material such as stainless steel or titanium alloy. The anchor body 4 is preferably circular in cross-section, and tapers from a maximum diameter near proximal end 8 to a minimum diameter toward distal end 16. Alternatively, the anchor body 4 may be formed of a translucent or transparent polymer material, and/or made of bioabsorbable materials such as polyglycolic or polylactic acid polymers. As used in the present application, the term "bioabsorbable" is considered to be interchangeable with the term "biodegradable," "resorbable," and "absorbable" to mean that the device can be absorbed by the body over time. Also, the measurements, angles and ratios between the dimensions of the suture anchor may be varied from those described above so as to be suitable for the conditions and applications in which the suture anchor is to be used.

Suture anchor 2 of the present invention is provided at proximal end 8 of body 4 with drive head 10 (preferably hexagonal) having a suture eye 12. The suture eye 12 may have various configurations and geometries. Preferably, suture eye 12 is in the form of an oval aperture in the drive head and holds at least one piece of suture, preferably braided suture. At the junction between the hex drive head 10 and the central body 4, the circumference of the central body 4 is advantageously larger than the outer circumferential dimension of the hex drive head 10. Accordingly, the enlarged body adjacent the hex drive head forms a hole sufficient to accommodate a hex driver disposed over the hex drive head. This allows the suture anchor 2 to be at least partially countersunk below the surface of the bone upon installation, by preventing impingement of the distal end of the hex driver on the bone surface.

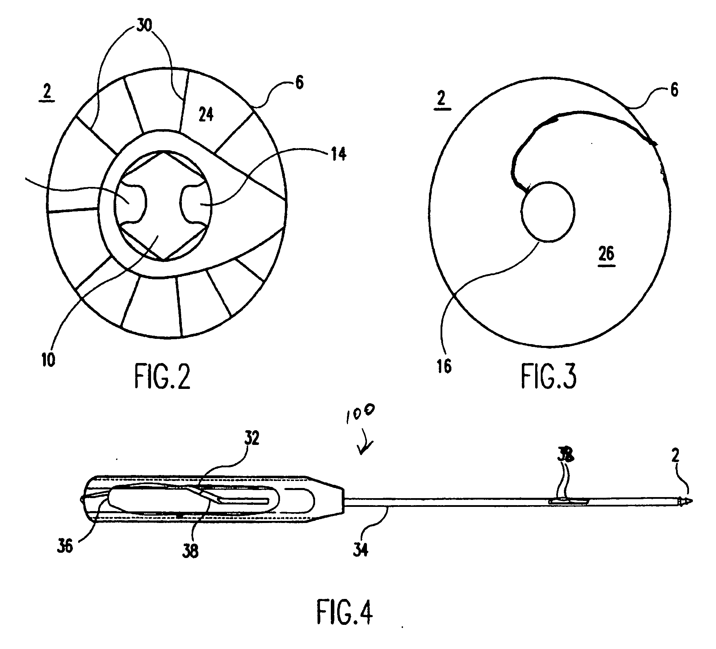

Channels 14 (FIG. 2) are formed along either side of the drive head 10 to accommodate the suture, for example, when the suture anchor is held in a suture anchor driver as described more fully below.

The central body 4 preferably tapers from the proximal end to terminate in a point 20 located at tip 18 of the distal end. The distal point 20 preferably is rounded to avoid possible breakage sometimes encountered when using a sharp point. As shown in FIG. 1, the rounded point 20 is approached by a concave, conically tapered surface 22. Conical taper 22 begins at the distal end of the screw thread, and features an angle of taper deeper than the taper of body 4.

Thread 6 illustrated in FIG. 1 has a proximal face 24, a distal face 26 and a break edge 28. Referring to FIG. 2, suture anchor 2 is shown from the proximal end. Radial ridge lines 30 are shown on proximal face 24 of corkscrew thread 6.

At the proximal end 8 of the anchor 2, the major (outside) diameter of the suture anchor thread of the present invention preferably is about 2.5 times the minor (inner) diameter of the thread, or the minor diameter of the body toward distal end 16. Accordingly, on a 5 mm diameter suture anchor, for example, where central core 4 is approximately 2 mm in diameter, the outer diameter of the thread is about 5 mm.

Preferably, between two and three flights or turns of thread 6 are provided along body 4, between proximal end 8 and distal end 16. The thickness of the thread increases proximally, such that at least the outer edge of the most proximal flight is thicker than the edge of each of the more distal flights, as shown in FIG. 1. Adjacent sections of each flight are separated by a gap that is determined by the number of turns per inch of the suture anchor thread. For example, a 5 mm suture anchor preferably has 6 turns per inch (per 25.4 mm), while a 3.5 mm suture anchor preferably has about 9. Accordingly, on a 5 mm or 6.5 mm suture anchor, the pitch distance from flight to flight is about 0.167 inches (4,242 mm). On a 3.5 mm suture anchor, pitch distance typically is about 0.118 inches (2,997 mm).

The pull-out strength and minimal tissue damage are enhanced by the relatively compressed cross-sectional aspect of the thread 6, particularly in relation to the broad axial faces of the threads. The distal and proximal faces of the threads preferably form a square or rounded break edge at the outer diameter of the thread. In addition, and as noted above, the thickness of the thread increases proximally, such that at least the most proximal flight is thicker than each of the more distal flights. Increased back-out resistance may be enhanced by surface features, such as radial ridges 30 provided on the top and/or bottom faces of the screw threads. The surface features augment the engagement between the thread surfaces and the surrounding tissue once the suture anchor is installed.

The present invention also provides a method of anchoring suture in bone using the suture anchor 2 of the present invention, the method not being part of the claimed invention. The method includes the steps of: (i) threading suture through the suture eye on the proximal end of the suture anchor 2 described above with reference to FIGS. 1-3; (ii) coupling a driver with the suture anchor 2; and turning the driver (with the suture anchor 2) to advance the suture anchor 2 into the bone.

The anchors of the present invention may be used for various arthroscopic procedures. Advantageously, the suture anchor can be installed using a hollow, cannulated grasper as described in U.S. Pat. No. 5,466,243 to Schmieding. The anchors are also advantageous for open and mini-open surgical procedures, such as open rotator cuff repair, as described in U.S. Pat. No. 5,575,801 to Habermeyer et al.

FIG. 4 illustrates suture anchor/driver assembly 100 including suture anchor 2 of FIGS. 1-3 threaded with two pieces of suture 32 and held on a cannulated suture anchor driver 34. Optionally, the suture anchor can be distributed with at least one strand of suture already threaded through the eye 12. For example, FIG. 4 illustrates suture strands 32 attached to the eye 12 and threaded through the cannula of driver 34, allowing the sutures to slide smoothly with minimal friction. In an exemplary embodiment, the suture strands 32 may be FiberWire composite sutures of alternating colors to maximize repair strength, aid in suture management and provide superior tying characteristics.

Suture strands 32 are threaded through the cannula of the driver 34 and secured on a hook and cleat 36 (FIG. 4) on the handle of the driver, to allow the distal end of the head of the driver to be inserted into the opening of the anchor 2 so that the suture anchor is driven into a pilot hole.

As shown in FIG. 4, the suture 32 is wrapped around cleat 36. Tension on the suture aids in retaining the suture anchor in the distal end of the driver. The suture may be held in place in slot 38 using a foam adhesive, for example, during shipping. Assembly 100 may be provided as a sterile unit ready for surgical application.

Sutures anchors according to the present invention can be used for arthroscopic procedures. The anchors are also advantageous for open and mini-open surgical procedures. Specific examples of applicable procedures include cortical bone-soft tissue fixation, Bankart and SLAP shoulder repairs.

A surgical method employing a threaded suture anchor with starting pitch, such as the threaded suture anchor 2 of FIGS. 1-4, generally includes preforming a hole for insertion of the anchor using a punch and a tap. The anchor is then engaged with a cannulated driver, such as driver 34 of FIG. 4, inserted into the pilot hole and turned to advance the suture anchor into the bone. The driver is then removed, and the suture extending through the internal suture eye 12 can then be manipulated and tied to secure soft tissue to bone.

Although the present invention has been described in relation to particular embodiments thereof, many other variations and modifications and other uses will become apparent to those skilled in the art. Therefore, the present invention is to be limited not by the specific disclosure herein, but only by the appended claims.

## Claims

1. A suture anchor (2) for securing suture to soft bone, comprising:
a central body (4) having a central axis, a distal end (16) and a proximal end (8);
a drive head (10) disposed on the proximal end of the central body (4), the drive head (10) including an eyelet (12) for receiving at least one strand of flexible material; and
a continuous thread (6) for anchoring the suture anchor (2) in soft bone, the continuous thread (6) being disposed in a spiral around the central body (4) and including a starting thread portion (7) at the distal end (16) of the central body (4) **characterized in, that** the starting thread portion (7) is oriented longitudinally relative to the central axis of the suture anchor (2) to facilitate insertion of the anchor (2).

2. The suture anchor (2) of claim 1, wherein the thread (6) has an inner diameter, an outer diameter and thickness, wherein the thickness of the thread at the outer edge of the thread increases proximally along a portion of the thread.

3. The suture anchor (2) of claim 2, wherein the outer diameter of the thread (6) is at least twice the inner diameter of the thread at the proximal end of the central body.

4. The suture anchor (2) of claim 1, further comprising a tip (18) disposed at the distal end of the central body (2).

5. The suture anchor (2) of claim 4, wherein the tip (18) has a tip taper which is greater than a taper of the central body (4).

6. The suture anchor (2) of claim 1, wherein the strand of flexible material is suture.

7. The suture anchor (2) of claim 2, wherein the ratio of the outer diameter to the inner diameter of the thread is between about 2.25 and about 2.75 along a portion of the thread.

8. The suture anchor (2) of claim 1, further comprising suture channels (14) located on the drive head (10).

9. The suture anchor (2) of claim 1, wherein the drive head (10) is hexagonal.

10. An assembly (100) of a driver and suture anchor for securing suture to soft bone, comprising:
a driver (34) for driving a suture anchor (2) into soft bone, the driver (34) comprising a tubular shaft having a distal end and a proximal end, and a handle disposed on the proximal end of the tubular shaft; and
a suture anchor (2) as claimed in claim 1 inserted into the distal end of the driver (34), the suture anchor central body (4) being tapered along a major portion thereof from a maximum diameter near the proximal end to a minimum diameter toward the distal end.

11. The suture anchor and driver assembly (100) of claim 10, further comprising a cleat (36) for holding the suture on an outer surface of the handle of the driver.

12. The suture anchor and driver assembly (100) of claim 11, wherein the cleat (34) includes a slot (38) for holding suture.

## Patentansprüche

1. Ein Nahtanker (2) zum Befestigen einer Naht an einen weichen Knochen, mit:
einem zentralkörper (4), der eine zentrale Achse, ein distales Ende (16) und ein proximales Ende (8) hat;
einem Antriebskopf (10), der am proximalen Ende des zentralkörpers (4) angeordnet ist, wobei der Antriebskopf (10) eine Öse (12) aufweist, zum Aufnehmen mindestens einer Faser aus flexiblem Material; und
mit einem kontinuierlichen Gewinde (6) zum Verankern des Gewindeankers (2) in einem weichen Knochen, wobei das durchgehende Gewinde (6) in einer Spirale um den zentralkörper (4) herum angeordnet ist und einen Startgewindeabschnitt (7) am distalen Ende (16) des zentralkörpers (4) aufweist, **dadurch gekennzeichnet, daß** der Startgewindeabschnitt (7) in Längsrichtung relativ zur zentralachse des Gewindenahtankers (2) ausgerichtet ist, um das Einsetzen des Ankers (2) zu erleichtern.

2. Der Nahtanker (2) nach Anspruch 1, bei dem das Gewinde (6) einen inneren Durchmesser, einen äußeren Durchmesser und eine Dicke hat, wobei die Dicke des Gewindes an der äußeren Kante des Gewindes längs eines Teiles des Gewindes in Proximalrichtung zunimmt.

3. Der Nahtanker (2) nach Anspruch 2, bei dem der Außendurchmesser des Gewindes (6) mindestens das Zweifache des inneren Durchmessers des Gewindes am proximalen Ende des Zentralkörpers beträgt.

4. Der Nahtanker (2) nach Anspruch 1, der weiterhin eine Spitze (18) aufweist, die am distalen Ende des zentralkörpers (2) angeordnet ist.

5. Der Nahtanker (2) nach Anspruch 4, bei dem die Spitze (18) eine Spitzenverjüngung aufweist, die größer ist als die Verjüngung des Zentralkörpers (4).

6. Der Nahtanker (2) nach Anspruch 1, bei dem die Faser aus flexiblem Material Nahtmaterial ist.

7. Der Nahtanker (2) nach Anspruch 2, bei dem das Verhältnis des äußeren Durchmessers zu dem inneren Durchmesser des Gewindes zwischen ca. 2,25 und ca. 2,75 längs eines Teiles des Gewindes ist.

8. Der Nahtanker (2) nach Anspruch 1, der weiterhin Nahtkanäle (14) enthält, die am Antriebskopf (10) angeordnet sind.

9. Der Nahtanker (2) nach Anspruch 1, bei dem der Antriebskopf (10) hexagonal ist.

10. Eine Anordnung (100) eines Antriebes und eines Nahtankers zum Verankern einer Naht an einem weichen Knochen, mit:
einem Antrieb (34) zum Eintreiben eines Nahtankers (2) in einen weichen Knochen, wobei der Antrieb (34) einen rohrförmigen Schaft mit einem distalen und einen proximalen Ende aufweist, und einem Handgriff, der am proximalen Ende des rohrförmigen Schaftes angeordnet ist; und
ein Nahtanker (2), wie im Anspruch 1 beansprucht, in das distale Ende des Antriebes (34) eingesetzt ist, wobei der zentralkörper (4) des Nahtankers längs seines Hauptteiles von einem maximalen Durchmesser nahe dem proximalen Ende zu einem minimalen Durchmesser in Richtung zum distalen Ende verjüngend ausgebildet ist.

11. Der Nahtanker und die Antriebsanordnung (100) nach Anspruch 10, die weiterhin eine Klammer (36) zum Halten der Naht an der äußeren Oberfläche des Handgriffes des Antriebes enthält.

12. Der Nahtanker und die Antriebsanordnung (100) nach Anspruch 11, bei der die Klemme (36) einen Schlitz (38) zum Halten der Naht aufweist.

## Revendications

1. Une attache-suture (2) pour attacher une suture à un os mou, comprenant :
un corps central (4) ayant un axe central, une extrémité distale (16) et une extrémité distale (8);
un actionneur (10) disposé sur l'extrémité proximale du corps central (4), l'actionneur comprenant (10) un oeillet (12) adapté pour recevoir au moins un cordon de matériau flexible; et
un fil continu (6) pour attacher l'attache-suture (2) dans l'os mou, le fil continu (6) étant disposé en spiral autour le corps central (4) et comprenant une première partie de fil (7) sur l'extrémité distale (16) du corps central (4), **caractérisée en ce que** la première partie de fil (7) est orienté longitudinalement par rapport à l'axe central de l'attache-suture (2) pour faciliter l'introduction de l'attache suture (2).

2. L'attache-suture (2) selon la revendication 1, dans lequel le fil (6) a un diamètre intérieur, un diamètre extérieur et une épaisseur, l'épaisseur du fil au bord extérieur du fil augmentant du côté proximal le long d'une partie du fil.

3. L'attache-suture (2) selon la revendication 2, dans lequel le diamètre extérieur du fil (6) est au moins deux fois le diamètre intérieur du fil à l'extrémité proximal du corps central

4. L'attache-suture (2) selon la revendication 1, comprenant aussi une tête (18) disposée à l'extrémité distale du corps central (2).

5. L'attache-suture (2) selon la revendication 4, dans lequel la tête (18) une conicité plus grande qu'une conicité du corps central (4)

6. L'attache-suture (2) selon la revendication 1, dans lequel le cordon de matériau flexible est une suture.

7. L'attache-suture (2) selon la revendication 2, dans lequel le rapport entre le diamètre extérieur et le diamètre intérieur du fil est entre environ 2,25 et environ 2,75 le long une partie du fil.

8. L'attache-suture (2) selon la revendication 1, comprenant aussi des canaux de suture (14) localisés sur l'actionneur (10)

9. L'attache-suture (2) selon la revendication 1, dans lequel l'actionneur (10) a une forme hexagonale.

10. Système (100) d'actionneur et attache-suture pour attacher la suture à un os mou, comprenant :
un actionneur (34) pour diriger l'attache-suture (2) dans l'os mou, l'actionneur (34) comprenant une tige tubulaire ayant une extrémité distale et une extrémité proximale, et
ayant une poigné disposée sur l'extrémité proximale de la tige tubulaire ; et
une attache-suture (2) selon la revendication 1 introduite dans l'extrémité distale l'actionneur (34), l'attache-suture comprenant un corps central (4) ayant une conicité le long d'une grande partie de celui-ci d'un diamètre maximal près de l'extrémité proximal à un diamètre minimal vers l'extrémité distale.

11. Système (100) d'actionneur et attache-suture selon la revendication 10, comprenant aussi une barrette (36) pour tenir la suture sur une surface extérieure de la poignée de l'actionneur.

12. Un système (100) d'actionneur et attache-suture selon la revendication 11, dans lequel la barrette (36) comprend une fente (38) pour tenir la suture.
